## Europäisches Patentamt

## European Patent Office

## Office européen des brevets

(19)

(11) Veröffentlichungsnummer: **0 171 603**
**B1**

# EUROPÄISCHE PATENTSCHRIFT

(12)

(45) Veröffentlichungstag der Patentschrift:
25.01.89

(21) Anmeldenummer: **85108582.9**

(22) Anmeldetag: **10.07.85**

(51) Int. Cl.⁴: **C 08 J 3/14,** C 08 L 67/04,
A 61 K 9/22

(54) Mikroporöse, pulverförmige Polylactide.

(30) Priorität: **03.08.84 DE 3428640**

(43) Veröffentlichungstag der Anmeldung:
**19.02.86 Patentblatt 86/8**

(45) Bekanntmachung des Hinweises auf die Patenterteilung:
**25.01.89 Patentblatt 89/4**

(84) Benannte Vertragsstaaten:
**CH DE FR GB LI NL**

(56) Entgegenhaltungen:
**EP-A-0 025 696**
**EP-A-0 094 536**
**EP-A-0 134 318**
**US-A-1 871 725**
**US-A-3 498 957**

**COLLOID & POLYMER SCIENCE, Band 261, Nr. 6,
1983, Seiten 477-484, Dr. Steinkopff Verlag,
Darmstadt, DE; S. GOGOLEWSKI u.a.: "Resorbable
materials of poly(L-lactide), III. Porous materials
for medical application"**

(73) Patentinhaber: **Akzo Patente GmbH, Postfach 10 01
49 Kasinostrasse 19- 23, D-5600 Wuppertal- 1 (DE)**

(72) Erfinder: **Lange, Wolfgang, Dr. Dipl.- Chem.,
Rosenstrasse 12, D-8753 Obernburg (DE)**

## Beschreibung

Die Erfindung betrifft mikroporöse, pulverförmige Polylactide, Verfahren zu deren Herstellung sowie derartige mikroporöse, pulverförmige Polylactide, welche in der Tier- und Humanmedizin wirksame Stoffe enthalten.

Polylactide sind bereits seit langem bekannt. Sie können als Homopolymere aber auch als Copolymere auf den verschiedensten Gebieten eingesetzt werden. Da Polylactide im allgemeinen biologisch abbaubar sind, insbesondere auch im menschlichen und tierischen Körper im Laufe der Zeit abgebaut werden, ist das Interesse an Formkörpern und auch an pulverförmigen Teilchen aus Polylactiden in jüngster Zeit stark gewachsen. Ein wichtiges Anwendungsgebiet sind mikroporöse, rieselfähige Polylactidpulver, die geeignet sind, größere Mengen an Wirkstoffen zu speichern und diese über längere Zeit hinaus an das umgebende Milieu abzugeben.

Es hat bisher nicht an Versuchen gefehlt, Polylactide in Form von Pulver herzustellen, das mikroporös ist und Wirkstoffe aufnehmen kann.

Unter Verwendung von bekannten Lösungsmitteln wie Benzol oder Toluol kann man zwar Polylactidlösungen herstellen, die unter Formgebung zu Formkörpern wie durchsichtigen Folien verarbeitbar sind; brauchbare Pulver, die mikroporös sind und Wirkstoffe speichern können, lassen sich unter Einsatz von Benzol und Toluol jedoch nicht erhalten.

In der DE-OS-3 218 151 wird ein Verfahren beschrieben, mikroporöse, pulverförmige Polylactide herzustellen, indem man Polylactid in Xylol auflöst, die entstandene klare Lösung abkühlt und das Xylol entfernt. Mit diesem Verfahren werden zwar mikroporöse Pulver erhalten, die in der Lage sind, Wirkstoffe zu speichern. Von Nachteil bei diesem Verfahren ist jedoch, daß das Material eine schuppenförmige, blattartige Struktur aufweist, die bei manchen Anwendungsgebieten von Nachteil ist. Von Vorteil wären Polylactidpulver mit abgerundeter, annähernd kugelförmiger Gestalt.

In der EP-A-134,318 werden Zusammensetzungen auf der Basis Polylactide beschrieben, welche den therapeutisch wirksamen Stoff Insulin enthalten.

Im Unterschied zu der vorliegenden Erfindung handelt es sich bei den in der EP-A-134,318 erwähnten Zusammensetzungen jedoch um Produkte, bei denen die Matrix aus Polylactiden als massive Struktur vorliegt, in der die Wirkstoffe wie Einlagerungen eingeschlossen sind. Die Abgabe der Wirkstoffe erfolgt über Diffusion.

Demgegenüber besitzen mikroporöse pulverförmige Polylactide gemäß der Erfindung ein mikroporöses Hohlraumvolumen, bei dem die einzelnen Mikroporen miteinander in Verbindung stehen. Aufgrund dieser speziellen Strukturen werden gemäß der Erfindung therapeutisch wirksame Stoffe im konvektiven Strom an das umgebende Milieu abgegeben.

Aufgabe der Erfindung ist es deshalb, mikroporöse, pulverförmige Polylactide zur Verfügung zu stellen, die die obengenannten Nachteile nicht aufweisen und eine abgerundete, annähernd kugelförmige Gestalt besitzen, die auf einfache Weise mit Wirkstoffen beladen werden können und in der Lage sind, diese Wirkstoffe über längere Zeit hinaus an die entsprechende Umgebung wieder abzugeben.

Aufgabe der Erfindung sind ferner mikroporöse, pulverförmige Polylactidteilchen, die sowohl mit geringeren als auch mit größeren Mengen von Wirkstoff beladen werden können, und die geeignet sind, in der Tier-und Humanmedizin Anwendung zu finden. Aufgabe der Erfindung ist ferner ein vorteilhaftes Verfahren zur Herstellung derartiger pulverförmiger Teilchen.

Diese Aufgabe wird durch ein Verfahren zur Herstellung von mikroporösen, pulverförmigen Polylactiden gelöst, das dadurch gekennzeichnet ist, daß man Polylactid durch Erwärmen in Phthalsäurediäthylester löst, die entstandene klare Lösung abkühlt und den Phthalsäurediäthylester entfernt. Vorzugsweise wird das Polylactid bei Temperaturen oberhalb 150°C gelöst. Nach dem Abkühlen kann der Phthalsäurediäthylester durch Absaugen und Extrahieren mit Äthanol vom Polylactidpulver entfernt werden. Vorzugsweise werden zur Herstellung der Lösung 15 bis 60 Teile Polylactid und 85 bis 40 Teile Phthalsäurediäthylester verwendet.

Es ist günstig, wenn man die Polylactidlösung zunächst mit einer Geschwindigkeit von 20°C bis 200°C pro Min. auf Zimmertemperatur abkühlt, das Gemisch eine Zeit lang stehenläßt, vorzugsweise etwa 90 Min. und sodann die entstandene Suspension filtriert. Als Polylactide können Polymere der dl-Milchsäure, 1(+)-Milchsäure, d(-)-Milchsäure allein oder in Kombination verwendet werden. Sehr geeignet sind Copolymere der erwähnten Milchsäuren mit weiteren Hydroxycarbonsäuren, wobei als weitere Hydroxycarbonsäure Glykolsäure bevorzugt ist.

Gegenstand der Erfindung sind auch microporöse, pulverförmige Polylactide, wie sie gemäß den vorstehend erläuterten Verfahren erhältlich sind.

Die mikroporösen pulverförmigen Polylactide, wie sie gemäß der Erfindung erhältlich sind, sind besonders zur kontrollierten Abgabe von therapeutisch wirksamen Stoffen in der Tier- und Humanmedizin geeignet, insbesondere für die intrakorporale, die subkutane und die intramuskuläre Abgabe von Wirkstoffen.

Unter Polylactiden im Sinne der Erfindung sind alle üblichen Polylactide zu verstehen, insbesondere die Homo- und Copolymeren der Milchsäuren, die als d(-)-Milchsäure, 1(+)-Milchsäure sowie als dl-Milchsäure verwendet werden können. Zur Herstellung von Copolymeren ist besonders die Glykolsäure geeignet.

Bevorzugt werden biologisch abbaubare Polylactide verwendet. Entsprechende Polylactide werden z. B. in Polymer 1979, Bd. 20, Seite 1459 - 1464 beschrieben. Auch in der Patentliteratur sind zahlreiche Hinweise zur Herstellung von Polylactiden zu finden. Als Beispiele seien genannt: EP-OS-2 6 599, US-PS-4 157 437, wo wiederum weitere Hinweise für die Herstellung von Polylactiden zu finden sind.

Die Herstellung der Lösung von Polylactid in Phthalsäurediäthylester kann an sich auf einfache Weise durchgeführt werden, z. B. durch Mischen der Komponenten und Erwärmen, vorzugsweise auf Temperaturen oberhalb 150°. Es ist empfehlenswert, das Polylactid in zerkleinerter Form einzusetzen und während des Lösens zu rühren. Nach kurzer Zeit entsteht eine klare Lösung. Falls Verunreinigungen vorhanden sind, die nicht lösbar sind, empfiehlt es sich, vor dem Abkühlen der Lösung das Gemisch zu filtrieren. Die heiße Lösung wird sodann langsam abgekühlt. Dies kann z. B. durch einfaches Stehenlassen der Lösung bei Zimmertemperatur geschehen. Es ist jedoch zweckmäßig, wenn die Abkühlung mit einer Geschwindigkeit von etwa 20°C bis 200°C pro Min. durchgeführt wird. Das abgekühlte Gemisch sollte sodann noch eine Zeit lang stehengelassen werden, zweckmäßigerweise etwa 90 Min. Je nach Konzentration und Eigenschaften des verwendeten Polylactids entsteht eine Suspension oder eine mehr oder weniger krümmelige Masse, die noch den Phthalsäurediäthylester enthält. Ein beachtlicher Teil des Phthalsäurediäthylesters kann durch Filtrieren bzw. Absaugen auf einer üblichen Nutsche vom Polylactid entfernt werden. Das zurückbleibende krümmelige Gut wird mit einer geeigneten Flüssigkeit, die Phthalsäurediäthylester löst, Polylactid hingegen nicht, extrahiert. Dazu ist insbesondere Äthanol geeignet. Als weitere Flüssigkeiten seien genannt: Isopropanol, Methanol u.dgl. Zum Extrahieren sind übliche Extraktionsvorrichtungen insbesondere der Soxhlet geeignet. Die noch anhaftende Extraktionsflüssigkeit kann durch einfaches Trocknen unter milden Bedingungen entfernt werden. Die erhaltene krümmelige Masse kann sodann in einfacher Weise, z. B. durch Passieren durch Sieb in die entsprechende Pulverform überführt werden. Ein Mahlen der Masse ist nicht erforderlich.

Es fallen pulverförmige Teilchen mit abgerundeter nahezu kugelförmiger Gestalt an, die rieselfähig sind und eine Größenverteilung mit ihrer Hauptmenge, d. h. ungefähr 75 bis 80 Gew.-% im Bereich von 100 bis 200 µm Teilchendurchmesser besitzen.

Die erhaltenen Pulver lassen sich aufgrund ihrer hervorragenden mikroporösen Struktur problemlos mit den verschiedensten Wirksubstanzen anfüllen. Sehr geeignet zum Einbringen der Wirksubstanzen sind wäßrige Lösungen, z. B. solche von Antibiotika, daß das Pulver hydrophilen Charakter besitzt und aufgrund der im Inneren der Pulver wirkenden Kapillarkräfte sich schnell mit der Wirkstofflösung vollsaugt. Durch Trocknen, z. B. unter reduziertem Druck ist es möglich, das als Lösungsmittel dienende Wasser schnell zu entfernen, so daß sich im Pulver ein Depot von festem Wirkstoff niederschlagen kann.

Durch Wahl der Konzentration der wäßrigen Lösung ist es möglich, den Gehalt an Wirkstoff im Pulver innerhalb weiter Grenzen zu variieren. Auch kann man nach Entfernen des Wassers das Pulver noch ein- oder sogar mehrmals mit wäßrigen Wirkstofflösungen behandeln, so daß es möglich ist, den Hohlraum im Pulver nahezu völlig mit Wirkstoff anzufüllen.

Da es auf diese Weise möglich ist, durch mehrmaliges Behandeln mit wäßrigen Wirkstofflösungen den Gehalt an Wirkstoff erheblich zu erhöhen und die erfindungsgemäßen Pulver sehr hohe Hohlraumvolumen besitzen können bis über 60 Vol.-%, maximal sogar bis etwa 85 Vol.-%, ist es gemäß der Erfindung möglich, eine Polylactidzusammensetzung zu erhalten, die bis zu etwa 85 Vol.-% an festem Wirkstoff enthält. Das Pulver kann somit ein Mehrfaches seines eigenen Gewichts an Wirkstoff einschließen.

Selbstverständlich können die mikroporösen Pulver auch mit nicht-wäßrigen Lösungen von Wirkstoffen gefüllt werden. Auch hier ist es möglich, das organische Lösungsmittel zu entfernen und die Menge an Wirkstoffen sehr hoch einzustellen.

Es ist auch möglich, Wirkstoffe, die selbst flüssig sind, ohne die Mitverwendung von Wasser oder nicht-wäßrigen Lösungsmitteln in das mikroporöse Material einzulagern.

Es war besonders überraschend, daß es gemäß der Erfindung möglich ist, mikroporöse rieselfähige Polylactidpulver herzustellen, die geeignet sind, größere Mengen an Wirkstoffen der verschiedensten Art, wie Medikamente, Polypeptide, Hormone, Antibiotika, Vitamine u.dgl. sowie Pesticide, Herbicide, Spurenelemente, Duftstoffe usw. zu speichern und über längere Zeit an die Umgebung wiederabzugeben. Da die microporösen Polylactidpulver selbst auch abbaubar sind und im z. B. menschlichen oder tierischen Körper zu ungiftigen Stoffen abgebaut werden können, sind die mikroporösen Pulver gemäß der Erfindung sehr vorteilhaft.

Das Polylactidpulver kann, wenn es mit Wirkstoff beladen ist, als solches eingesetzt werden. Es kann auch z. B. durch Verpressen zu Formkörpern wie Tabletten oder dünnen Stäben weiterverarbeitet werden. Selbstverständlich ist es möglich, aus dem Polylactidpulver zunächst den entsprechenden Formkörper herzustellen und diesen dann mit Wirkstoff zu beladen. In einer vorteilhaften Ausführungsform werden mit festem oder flüssigen Wirkstoff beladene Polylactidpulver gemäß der Erfindung in einer Matrix eines abbaubaren Polymers verteilt. Auf diese Weise ist es möglich, weitere Kombinationen mit den verschiedensten Abbaugeschwindigkeiten und controlled release-Eigenschaften herzustellen. Die Herstellung derartiger Kombinationen wird in der DE-OS-3 218 150 beschrieben, auf deren Offenbarung sich hier ausdrücklich bezogen wird.

Polylactidpulver, die mit geeigneten Wirkstoffen beladen sind, können insbesondere in der Human- und in der Tiermedizin eingesetzt werden; sie können intrakorporal, subkutan oder intramuskulär durch Injektion verwendet werden.

Das mikroporöse Polylactidpulver gibt dann im tierischen oder im menschlichen Körper im Laufe der Zeit seinen Wirkstoff ab und wird nach bestimmter Zeit selbst zu ungiftigen Produkten vom Organismus abgebaut.

Es ist auch möglich, Formkörper, wie kleine Stöbchen operativ an bestimmten Stellen des Körpers z. B. unter die Haut, z. B. ins Fettgewebe einzuführen.

Auch in der Land und Forstwirtschaft, z. B. bei der Bekämpfung von Schädlingen, wie Insekten kann Polylactidpulver gemäß der Erfindung mit großem Vorteil eingesetzt werden.

Da die chemische Zusammensetzung des Polymers in weiten Grenzen variiert werden kann, so daß die Abbaubarkeit und die Abbaugeschwindigkeit des Polymeren an das jeweils vorhandene Milieu angepaßt werden kann, da die Menge und die Art des Wirkstoffes in nahezu beliebiger Weise variiert werden kann, lassen sich die erfindungsgemäßen mikroporösen Polylactidpulver auch sehr vielseitig einsetzen. Selbstverständlich sind auch Kombinationen von verschiedenen Wirkstoffen möglich.

Die Erfindung wird durch folgende Beispiele näher erläutert:

1.) Herstellung des Polylactidpulvers

300 g 1-Polylactid(Homopolymer der 1-Milchsäure) werden mit 76,3 Gew.-% Phthalsäurediäthylester bei 160°C ca. 90 Min lang unter Rühren gelöst. Die klare Lösung wurde anschließend auf Bleche zu 2 bis 5 mm dicken Schichten ausgegossen. Nach weiteren 90 Min. wird die entstandene Suspension abgenutscht und mit Äthanol mehrfach gewaschen.

Nach 3 Std. Trocknung im Vakuumtrockner bei 50°C wurde ein weißes rieselfähiges Pulver folgender Teilchenverteilung gewonnen.

| Fraktion (µm) | Anteil (%) |
|---|---|
| >400 | 0 |
| 400-315 | 0 |
| 315-200 | 14,58 |
| 200-100 | 78,03 |
| 100-80 | 5,73 |
| 80-50 | 0 |
| <50 | 1,65 |
| | 100 |

Zur Beladung mit Wirkstoff wurden 20 g 1-Polylactidpulver unter Rühren bei Zimmertemperaturen mit 7 ml einer 800 mg Acetylsalicylsäure enthaltenden äthanolischen Lösung versetzt. Nach 10-minütiger Zugabe der Lösung bei Zimmertemperatur wurde das Pulverlösungsmittelgemisch noch 30 Min gerührt. Dann wurden 60 Min. lang 30 l $N_2$/h zur Trocknung über das Pulver geleitet. Nach 5-maliger Wiederholung dieses Vorgangs hatte das Pulver einen Beladungsgrad von ca. 20 % Acetylsalicylsäure.

## Patentansprüche

1. Verfahren zur Herstellung von mikroporösen pulverförmigen Polylactiden, dadurch gekennzeichnet, daß man Polylactid durch Erwärmen in Phthalsäurediäthylester löst, die entstandene klare Lösung abkühlt und den Phthalsäurediäthylester entfernt.

2. Verfahren nach Anspruch 1, dadurch gekennzeichnet, daß man das Polylactid oberhalb 150°C löst.

3. Verfahren nach einem der Ansprüche 1 bis 2, dadurch gekennzeichnet, daß man den Phthalsäurediäthylester durch Absaugen und Extrahieren mit Äthanol entfernt.

4. Verfahren nach einem der Ansprüche 1 bis 3, dadurch gekennzeichnet, daß man zur Herstellung der Lösung 15 bis 60 Teile Polylactid und 85 bis 40 Teile Phthalsäurediäthylester verwendet.

5. Verfahren nach einem der Ansprüche 1 bis 4, dadurch gekennzeichnet, daß man die Polylactidlösung zunächst mit einer Geschwindigkeit von 20°C bis 200°C pro Min. auf Zimmertemperatur abkühlt und nach etwa 90 weiteren Minuten die entstandene Suspension filtriert.

6. Verfahren nach einem der Ansprüche 1 bis 5, dadurch gekennzeichnet, daß man als Polylactid Polymere der dl-Milchsäure, 1(+)-Milchsäure, d(-)-Milchsäure allein oder in Kombination verwendet werden.

7. Verfahren nach einem der Ansprüche 1 bis 6, dadurch gekennzeichnet, daß man Copolymere der Milchsäure und weiterer Hydroxycarbonsäuren verwendet.

8. Verfahren nach Anspruch 7, dadurch gekennzeichnet, daß man als weitere Hydroxycarbonsäure Glykolsäure verwendet.

9. Mikroporöse, pulverförmige Polylactide, erhältlich nach einem Verfahren gemäß einem der Ansprüche 1 bis 8.

10. Mikroporöse, pulverförmige Polylactide nach Anspruch 9 enthaltend in der Tier- und Humanmedizin therapeutisch wirksame Stoffe.

11. Mikroporöse, pulverförmige Polylactide nach Anspruch 10, enthaltend in der Tier- und Humanmedizin therapeutisch wirksame Stoffe für die intrakorporale Abgabe.

12. Mikroporöse, pulverförmige Polylactide nach Anspruch 10, enthaltend in der Tier- und Humanmedizin therapeutisch wirksame Stoffe für die subkutane Abgabe.

13. Mikroporöse, pulverförmige Polylactide nach Anspruch 10, enthaltend in der Tier- und Humanmedizin therapeutisch wirksame Stoffe für die intramuskuläre Abgabe.


## Claims

1. A process for the production of microporous powder-form polylactides, characterized in that polylactide is dissolved in phthalic acid diethyl ester by heating, the clear solution formed is cooled and the phthalic acid diethyl ester is removed.

2. A process as claimed in claim 1, characterized in that the polylactide is dissolved above 150°C.

3. A process as claimed in claim 1 or 2, characterized in that the phthalic acid diethyl ester is removed by filtration under suction and extraction with ethanol.

4. A process as claimed in any of claims 1 to 3, characterized in that 15 to 60 parts polylactide and 85 to 40 parts phthalic acid diethyl ester are used to prepare the solution.

5. A process as claimed in any of claims 1 to 4, characterized in that the polylactide solution is first cooled to room temperature at a rate of 20 to 200°C per minute and the suspension formed is filtered after about another 90 minutes.

6. A process as claimed in any of claims 1 to 5, characterized in that polymers of dl-lactic acid, l(+)-lactic acid, d(-)-lactic acid are used individually or in combination as the polylactide.

7. A process as claimed in any of claims 1 to 6, characterized in that copolymers of lactic acid and other hydroxycarboxylic acids are used.

8. A process as claimed in claim 7, characterized in that glycolic acid is used as the other hydroxycarboxylic acid.

9. Microporous, powder-form polylactides obtainable by the process claimed in any of claims 1 to 8.

10. Microporous, powder-form polylactides as claimed in claim 9 containing substances of therapeutic value in veterinary and human medicine.

11. Microporous, powder-form polylactides as claimed in claim 10 containing substances of therapeutic value in veterinary and human medicine intended for intracorporal administration.

12. Microporous, powder-form polylactides as claimed in claim 10 containing substances of therapeutic value in veterinary and human medicine intended for subcutaneous administration.

13. Microporous, powder-form polylactides as claimed in claim 10 containing substances of therapeutic value in veterinary and human medicine intended for intramuscular administration.


## Revendications

1. Procédé de préparation de polylactides sous forme pulvérulente et microporeuse, caractérisé en ce que l'on dissout le polylactide par chauffage dans du phtalate de diéthyle, on refroidit la solution limpide qui se forme, et l'on élimine le phtalate de diéthyle.

2. Procédé conforme à la revendication 1, caractérisé en ce que l'on dissout le polylactide au-dessus de 150°C.

3. Procédé conforme à l'une des revendications 1 à 2, caractérise en ce que l'on élimine le phtalate de diéthyle par filtration à la trompe et extraction à l'éthanol.

4. Procédé conforme à l'une des revendications 1 à 3, caractérisé en ce que l'on utilise, pour la préparation de la solution, de 15 à 60 parties de polylactide et de 85 à 40 parties de phtalate de diéthyle.

5. Procédé selon l'une des revendications 1 à 4, caractérisé en ce que l'on refroidit d'abord la solution de polylactide à une vitesse de 20°C à 200°C par minute, jusqu'à la température ambiante, et on filtre la suspension formée après environ 90 minutes supplémentaires.

6. Procédé conforme à l'une des revendications 1 à 5, caractérisé en ce que l'on utilise comme polylactide des polymères de l'acide dl-lactique, de l'acide 1(+)-lactique, de l'acide d(-)- lactique, seuls ou en combinaison.

7. Procédé conforme l'une des revendications 1 à 6, caractérisé en ce que l'on utilise des copolymères de l'acide lactique et d'autres acides hydroxycarboxyliques.

8. Procédé conforme à la revendication 7, caractérisé en ce que l'on utilise de l'acide glycolique comme autre acide hydroxycarboxylique.

9. Polylactide sous forme pulvérulente et microporeuse, obtenu selon un procédé conforme à l'une des revendications 1 à 8.

13. Polylactide sous forme pulvérulente et microporeuse, conforme à la revendication 9, contenant des substances thérapeutiquement actives en médecine humaine et vétérinaire.

11. Polylactide sous forme pulvérulente et microporeuse, conforme à la revendication 10, contenant des substances thérapeutiquement actives en médecine humaine et vétérinaire pour administration intracorporelle.

12. Polylactide sous forme pulvérulente et microporeuse, conforme à la revendication 10, contenant des matières thérapeutiquement actives en médecine humaine et vétérinaire pour administration sous-cutanée.

13. Polylactide sous forme pulvérulente et microporeuse, conforme à la revendication 10, contenant des substances thérapeutiquement actives en médecine humaine et vétérinaire pour administration intramusculaire.